(19) Europäisches Patentamt

European Patent Office

Office européen des brevets

(11) **EP 1 016 641 A1**

(12) **EUROPÄISCHE PATENTANMELDUNG**

(43) Veröffentlichungstag:
05.07.2000  Patentblatt 2000/27

(51) Int. Cl.$^7$: **C07C 5/333**, B01J 23/62

(21) Anmeldenummer: **99125259.4**

(22) Anmeldetag: **17.12.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **18.12.1998 DE 19858747**

(71) Anmelder:
**Linde Aktiengesellschaft**
**65189 Wiesbaden (DE)**

(72) Erfinder:
• **Fritz, Peter-Matthias, Dr. Dipl.-Chem.**
  **82008 Unterhaching (DE)**
• **Bölt, Heinz, Dipl.-Ing.**
  **82515 Wolfratshausen (DE)**

(74) Vertreter: **Kasseckert, Rainer**
**Linde Aktiengesellschaft,**
**Zentrale Patentabteilung**
**82049 Höllriegelskreuth (DE)**

(54) **Verfahren und Katalysatorstation zur Dehydrierung von Alkanen**

(57)    Die Erfindung betrifft ein Verfahren und eine Katalysatorstation zur katalytischen Dehydrierung von Alkanen einer Kettenlänge von 2 bis 10 Kohlenstoffatomen, wobei einem die Alkane enthaltenden Einsatz ein Verdünnungsgas zugemischt, der Mischstrom unter dehydrierenden Bedingungen über eine Katalysatorstrecke mit Katalysatorpartikeln geleitet wird und die Alkane im Mischstrom mindestens teilweise dehydriert und dabei hauptsächlich in Alkene gleicher Kohlenstoffkettenlänge wie die Alkane umgesetzt werden. Erfindungsgemäß wird das Verdünnungsgas mit Wasserdampf und Wasserstoff gebildet und der Mischstrom auf der Katalysatorstrecke isotherm geführt. Der die Dehydrierung begünstigende Katalysator enthält Platin und Zinn auf einem Hydrotalcit-Träger und wird in Partikelform in der Katalysatorstrecke verwendet.

Bl.

**Figur**

EP 1 016 641 A1

**Beschreibung**

Die Erfindung betrifft ein Verfahren zur katalytischen Dehydrierung von Alkanen einer Kettenlänge von 2 bis 10 Kohlenstoffatomen, wobei einem die Alkane enthaltenden Einsatz ein Verdünnungsgas zugemischt, der Mischstrom unter dehydrierenden Bedingungen über eine Katalysatorstrecke mit Katalysatorpartikeln geleitet wird und die Alkane im Mischstrom mindestens teilweise dehydriert und dabei hauptsächlich in Alkene gleicher Kohlenstoffkettenlänge wie die Alkane umgesetzt werden.

Die Erfindung betrifft außerdem eine Katalysatorstation mit mindestens zwei Reaktoren zur katalytischen Dehydrierung von Alkanen, wobei jeder Reaktor parallel geschaltete Rohre mit Katalysatorschüttungen enthält und die parallel geschalteten Rohre in einem Feuerraum mit Deckenbefeuerung angeordnet sind.

Bekannt ist die Herstellung von Propen aus einem Kohlenwasserstoffeinsatz in einem Steamcrakker. Sie ist in einem bestimmten Verhältnis an die Ethenproduktion aus dem Kohlenwasserstoffeinsatz gekoppelt. Der stärker als der Ethenverbrauch steigende Propenverbrauch erfordert jedoch eine Entkoppelung der Propen- und der Ethenproduktion.

Die Entkoppelung wird durch Einführung von Verfahren zur Dehydrierung von Alkanen ermöglicht. Ein solches Verfahren ist z.B. in der Veröffentlichung: H. Bölt u.a. "Dehydrogenation of propane/butane" in Linde Reports on Science and Technology No. 49 (1991) beschrieben. Die endotherme Dehydrierung wird in einem Reaktor vom "Reformer-Typ" mit Deckenbefeuerung durchgeführt. Mit einem solchen Reaktor ist ein nahezu isothermer Betrieb möglich. Eine konstante Konversion und Produktzusammensetzung, wie sie insbesondere im Zerlegungsteil nach dem Reaktor zur Gewinnung der reinen Produkte in einem technischen Betrieb erforderlich ist, wird durch sukzessive Erhöhung der Temperatur des Reaktors erreicht. Im Verlauf von 6 Stunden kann so ein Dehydrierbetrieb ohne Abnahme der Konversion und der Selektivität der Reaktion in Richtung der gewünschten Alkene erzielt werden, ohne den Reaktor in dieser Zeit zu regenerieren. Die gewünschte Konversion und Selektivität werden in diesem Verfahren mit Hilfe eines in der Veröffentlichung nicht genannten Chrom-Katalysators ohne Wasserdampfverdünnung und meist ohne Wasserstoffverdünnung des Einsatzes erreicht. Daten zur Konversion und Selektivität werden nicht genannt.

Aus der Patentschrift US 4 902 849 ist ein Verfahren bekannt, das einen Katalysator mit aus Metallen der Gruppe IIA und IIB gebildetem Aluminat, mit Metallverbindungen der Gruppe IVA und mit Metallen der Gruppe VIII des Periodensystems der Elemente enthält. In einem Beispiel zu diesem Verfahren wird gezeigt, daß mit einem $ZnO/Al_2O_3/SnO_2/Pt$-Katalysator während einer Laufzeit von 6,7 Stunden und mit einer hohen Wasserdampfverdünnung von etwa 4 mol/mol die Isobutan-Konversion von 58,2 auf 50,8% abnimmt und die Selektivität der Umsetzung zu Isobuten von 84,4% auf 92,0% zunimmt. Erwünscht sind jedoch eine bessere Selektivität und Konversion, sowie eine niedrigere Dampfverdünnung.

Aufgabe der Erfindung ist es daher, die Konversion und Selektivität der Dehydrierung bei niedriger Wasserdampfverdünnung zu erhöhen und eine wirtschaftlichere Herstellung der Alken-Produkte zu ermöglichen.

Diese Aufgabe wird erfindungsgemäß gelöst von einem Verfahren mit dem Merkmalen des Anspruchs 1 und von einer Katalysatorstation mit den Merkmalen des Anspruchs 16. Ausführungen der Erfindung sind Gegenstand von Unteransprüchen.

Kennzeichnend an dem erfindungsgemäßen Verfahren ist, daß das Verdünnungsgas mit Wasserdampf und Wasserstoff gebildet wird, der Mischstrom auf der Katalysatorstrecke isotherm geführt wird und der die Dehydrierung begünstigende Katalysator mit Platin und Zinn auf einem Hydrotalcit-Träger gebildet und in Partikelform in der Katalysatorstrecke verwendet wird.

Die Verdünnung ermöglicht die Einstellung eines gegenüber dem Verfahrensdruck erniedrigten Alkanpartialdruckes mit einem günstigen Gleichgewicht für die Umsetzung. Die Verwendung von Wasserdampf und Wasserstoff führt zu langen Betriebszeiten mit dem verwendeten Katalysator, da im Vergleich zum Stand der Technik weniger Koks auf dem Katalysator abgeschieden wird, da dieser zum größten Teil gemäß der Reaktion

$$C + H_2O \rightarrow CO + H_2$$

vergast wird.

Im Einsatz können als die Alkane Butan und Propan oder deren Gemische und als Butan bevorzugt Isobutan verwendet werden. Der erfindungsgemäße Katalysator ist für diese Einsatzarten entwickelt worden und deshalb für diese besonders geeignet.

In dem mit dem Wasserdampf, mit dem Wasserstoff und mit den Alkanen gebildeten Mischstrom kann gemessen an den eingesetzten Alkanen ein Wasserdampfanteil von 0,5 bis 2 mol/mol und ein Wasserstoffanteil von 5 bis 25 mol% verwendet werden. Die unerwünschte Koksbildung beträgt dann weniger als 0,05 Gewichtsprozent der eingesetzten Alkane.

Zur Einstellung von dehydrierenden Bedingungen kann auf der Katalysatorstrecke ein Druck zwischen Atmosphärendruck und 10 bar, vorzugsweise zwischen Atmosphärendruck und 1,3 bar verwendet werden. Zu niedrigen Drücken hin wird das Reaktionsgleichgewicht zum Alken verschoben und damit die Alkenausbeute erhöht. Indem Unterdruck vermieden wird, können Leckagen von Luft in den Reaktionsraum hinein verhindert werden.

Zur Einstellung von dehydrierenden Bedingungen kann auf der Katalysatorstrecke eine Temperatur zwischen 570 und 650°C, vorzugsweise zwischen 580 und 620°C verwendet werden. Bei einer hohen Konversion, z.B. von 40 bis 60% Propan zu Propen, tritt nur sehr geringe Koksbildung auf und es werden lange Betriebszeiten ohne Regenerierung erreicht.

Zur Einstellung von dehydrierenden Bedingungen auf der Katalysatorstrecke kann eine Raumgeschwindigkeit zwischen 500 und 2000 h$^{-1}$ vorzugsweise zwischen 800 und 1100 h$^{-1}$ verwendet werden.

Die dehydrierenden Bedingungen werden mit Vorteil über eine Laufzeit zwischen 6 und 100 h, vorzugsweise zwischen 30 und 80 Stunden beibehalten ohne zu regenerieren.

Die Katalysatorstrecke wird am besten zeitlich vor und nach der Dehydrierlaufzeit regeneriert.

Für einen kontinuierlichen Betrieb über die Laufzeit der Katalysatorstrecke hinaus kann mindestens eine weitere Katalysatorstrecke verwendet werden, wobei im zyklischen Wechsel ständig mindestens eine Katalysatorstrecke unter dehydrierenden Bedingungen betrieben wird und dabei eine weitere Katalysatorstrecke regeneriert wird.

Für den kontinuierlichen Betrieb können günstig insgesamt drei Reaktorstrecken verwendet werden, wobei im zyklischen Wechsel ständig zwei Katalysatorstrecken unter dehydrierenden Bedingungen betrieben werden und dabei eine dritte Katalysatorstrecke regeneriert wird.

Das Regenerieren der Katalysatorstrecke kann durch Abbrennen von während der Dehydrierung gebildetem Koks vorgenommen werden, indem ein Regeneriergas über die Katalysatorstrecke geleitet wird, wobei das Regeneriergas durch gleitend oder in Stufen erhöhte Sauerstoffzufuhr, beispielsweise durch Zuspeisung von Luft oder Sauerstoff, zu Wasserdampf und/oder zu einem Inertgas wie z.B. Stickstoff gebildet wird, und indem in einem nachfolgenden Schrift Wasserstoff oder eine Mischung mit Wasserstoff und mit Alkanen über die Katalysatorstrecke geleitet wird, wobei z.B. Alkane wie im Einsatz verwendet werden. Dadurch wird eine Reduktion der Katalysatoroberfläche für einen nächsten Dehydrierschritt erzielt.

Im folgenden steht $C_x$ für Kohlenstoffverbindungen mit einer Kettenlänge von x Kohlenstoffatomen im Molekül, $C_{x+}$ (bzw. $C_{x-}$) für Moleküle mit x oder mehr als x (bzw. x oder weniger als x) Kohlenstoffatomen.

In dem die Alkane enthaltenden Einsatz kann Propan verwendet, in der Katalysatorstrecke hauptsächlich zu Propen umgesetzt, als aus der Katalysatorstrecke austretender Rohpropenstrom verdichtet und in einer $C_2/C_4$-Trennsäule rektifikatorisch in ein $C_3$-Kopfprodukt und ein $C_{3+}$-Sumpfprodukt zerlegt werden und anschließend das $C_3$-Kopfprodukt durch Tieftemperaturrektifikation (TTR) in eine $C_3$-Fraktion und eine hauptsächlich Wasserstoff enthaltende Fraktion zerlegt werden.

Bei der Umsetzung von Propan zu Propen wird mit der $C_2/C_4$-Trennung vor der TTR erreicht, daß $C_3$ zu einem großen Teil in das Sumpfprodukt gelangt und dadurch an der TTR vorbeigeführt wird, wodurch die aufwendige TTR wesentlich entlastet wird und kleiner ausgelegt werden kann.

Das $C_{3+}$-Sumpfprodukt wird mit Vorteil rektifikatorisch in eine $C_{4+}$-Fraktion und eine zweite $C_3$-Fraktion zerlegt.

Die erste und die zweite $C_3$-Fraktion können dann einem einzigen $C_3$-Splitter zugeführt und in eine Propenfraktion und eine Propanfraktion getrennt werden.

Alternativ kann das $C_{3+}$-Sumpfprodukt dem $C_3$-Splitter zugeführt werden und die Abtrennung der $C_{4+}$-Komponenten aus dem Sumpfprodukt des $C_3$-Splitters erfolgen.

Die Propenfraktion kann als reines Propenprodukt einer weiteren Verwendung zugeführt werden. Die gewonnene Propanfraktion kann mit Vorteil vor die Katalysatorstrecke zurückgeführt und im Einsatz verwendet werden.

Kennzeichnend an der erfindungsgemäßen Katalysatorstation ist, daß die Katalysatorschüttungen Katalysatorpartikel mit Platin und Zinn auf einem Hydrotalcit-Träger enthalten.

Die Erfindung wird anhand einer Ausführungsform mit einer Figur näher erläutert.

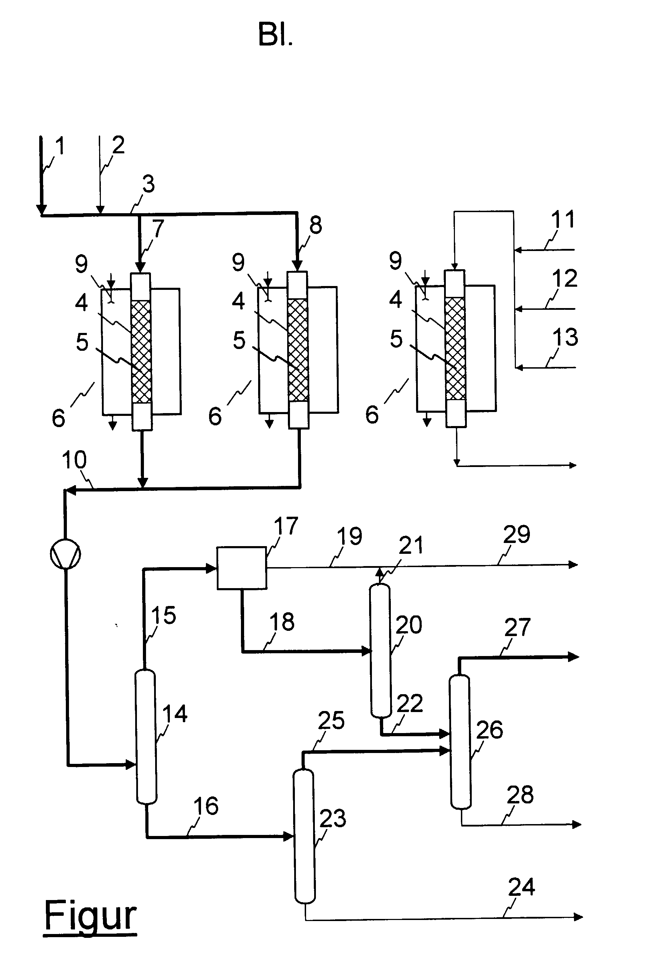

Die Figur zeigt schematisch eine Ausführung des erfindungsgemäßen Verfahrens mit Katalysatorstation 1 bis 13, wobei der Trennteil 14 bis 29 nach der Katalysatorstation typisch für eine Anwendung des erfindungsgemäßen Verfahrens in einer Anlage zur Propengewinnung ausgestaltet ist. Einem Propan enthaltenden Einsatz 1 wird ein Verdünnungsgas 2 zugemischt, das Dampf und Wasserstoff enthält. Der Mischstrom 3 wird bei einer Temperatur knapp unter 600°C in eine Katalysatorstation mit drei Reaktoren 6 geleitet, die in den Katalysatorrohren 4 Katalysatorschüttungen 5 mit einem Platin/Zinn/Hydrotalcit-Katalysator in Form von Katalysatorpartikeln enthalten. Die Reaktoren 6 sind mit einem deckenbefeuerten Feuerraum mit Brennern 9 ausgestattet. Der Mischstrom 3 wird auf zwei Ströme 7 und 8 aufgeteilt und unter dehydrierenden Bedingungen parallel über zwei der drei Reaktoren 6 geleitet. Die insbesondere auf dem ersten Drittel der Katalysatorstrecke (in der Figur im oberen Drittel der Katalysatorschüttung 5 des Reaktors 6 nahe dem Eintritt des Mischstromes 3 in die Schüttung 5) benötigte Wärmezufuhr und isotherme Reaktionsführung wird durch den deckenbefeuerten Feuerraum mit geregelten Brennern 9 auf einfache Weise und genau genug ermöglicht. Die Ströme 7 und 8 werden nach der Reaktion und weitgehenden Konversion des enthaltenen Propan zu Propen zu einem Rohpropenstrom 10 zusammengeführt und in den Trennteil 14 bis 29 geleitet.

Während zwei der drei Reaktoren dehydrierend betrieben werden, wird ein dritter Reaktor regeneriert, indem erst ein Regeneriergas, das durch Zufuhr von Inertgas 11 und Luft 12 gebildet wird, durch den Reaktor 6 geleitet wird, um beim Dehydrierbetrieb gebildeten Koks abzubrennen, und in einem späteren Schritt ein wasserstoffhaltiges Gas 13 durch den Reaktor geleitet wird, um die Oberfläche der Katalysatorpartikel zu hydrieren.

Der in den Trennteil geleitete Rohpropenstrom 10 wird verdichtet und in einer $C_2/C_4$ Trennsäule 14 rektifikatorisch in ein $C_3$-Kopfprodukt 15 und ein $C_{3+}$-Sumpfprodukt 16 zerlegt. Das Kopfprodukt 15 wird durch stufenweise Kondensation 17 in eine $C_2/C_3$-Fraktion 18 und eine hauptsächlich Wasserstoff enthaltende Fraktion 19 zerlegt. Die Fraktion 18 wird in einer $C_2/C_3$-Trennsäule 20 in eine $C_2$-Fraktion 21 und eine erste $C_3$-Fraktion 22 zerlegt.

Das $C_{3+}$-Sumpfprodukt 16 wird in einer $C_3/C_4$-Trennsäule 23 in eine $C_{4+}$-Fraktion 24 und eine zweite $C_3$-Fraktion 25 zerlegt. Die erste $C_3$-Fraktion 22 und die zweite $C_3$-Fraktion 25 werden einem $C_3$-Splitter 26 zugeführt und in ein Propenprodukt 27 und eine Propanfraktion 28 getrennt.

Die Fraktion 19 und die Fraktion 21 können zusammengeführt und als Brenngas 29 verwendet werden. Die Propanfraktion 28 kann einer Verwendung zugeführt, insbesondere rezykliert und im Propaneinsatz genutzt werden.

Beispiel:

Eine Propandehydrieranlage wird mit drei Reaktoren von jeweils 50% Anlagenkapazität betrieben. Mit einem Dehydriertakt von 75h, einer $H_2$-Verdünnung von 12 mol% und einer Dampfverdünnung von 1:1 Molanteilen (Mole jeweils im Vergleich zum eingesetzten Propan) und mit einem Betrieb bei 1,2 bar, 590°C und einer Raumgeschwindigkeit von 700 h$^{-1}$ werden folgende Ergebnisse erzielt:

| | |
|---|---|
| Propankonversion | > 50 Prozent |
| Propenselektivität | > 97 Gewichtsprozent |
| Koksbildung | < 0,05 Gewichtsprozent |

Wie das Beispiel zeigt, werden mit der Erfindung in einer Anlage zur Dehydrierung von Alkanen im Vergleich zum Stand der Technik folgende Vorteile erzielt: Die Dampfverdünnung ist niedriger als beispielsweise im Verfahren nach US 4 902 849. Trotzdem sind die Konversion und die Selektivität höher. Als Folge hiervon ist der Anlagendurchsatz für eine vorgegebene Produktmenge kleiner, die ganze Anlage kann kleiner ausgeführt werden und ist mit weniger Propaneinsatz, geringerem Betriebsmittelverbrauch und geringeren Investitionskosten wirtschaftlicher als eine Anlage nach dem Stand der Technik.

**Patentansprüche**

1. Verfahren zur katalytischen Dehydrierung von Alkanen einer Kettenlänge von 2 bis 10 Kohlenstoffatomen, wobei einem die Alkane enthaltenden Einsatz ein Verdünnungsgas zugemischt, der Mischstrom unter dehydrierenden Bedingungen über eine Katalysatorstrecke mit Katalysatorpartikeln geleitet wird und die Alkane im Mischstrom mindestens teilweise dehydriert und dabei hauptsächlich in Alkene gleicher Kohlenstoffkettenlänge wie die Alkane umgesetzt werden, **dadurch gekennzeichnet**, daß das Verdünnungsgas mit Wasserdampf und Wasserstoff gebildet wird, der Mischstrom auf der Katalysatorstrecke isotherm geführt wird und der die Dehydrierung begünstigende Katalysator mit Platin und Zinn auf einem Hydrotalcit-Träger gebildet und in Partikelform in der Katalysatorstrecke verwendet wird.

2. Verfahren nach Anspruch 1, dadurch gekennzeichnet, daß im Einsatz als die Alkane Butan oder Propan oder deren Gemische und als Butan bevorzugt Isobutan verwendet werden.

3. Verfahren nach Anspruch 1 oder 2, dadurch gekennzeichnet, daß in dem mit dem Wasserdampf, mit dem Wasserstoff und mit den Alkanen gebildeten Mischstrom gemessen an den eingesetzten Alkanen ein Dampfanteil von 0,5 bis 2,0 mol/mol und ein Wasserstoffanteil von 5 bis 25 mol% verwendet werden.

4. Verfahren nach einem der Ansprüche 1 bis 3, dadurch gekennzeichnet, daß zur Einstellung von dehydrierenden Bedingungen auf der Katalysatorstrecke Drücke zwischen Atmosphärendruck und 10 bar, vorzugsweise zwischen Atmosphärendruck und 1,3 bar verwendet werden.

5. Verfahren nach einem der Ansprüche 1 bis 4, dadurch gekennzeichnet, daß zur Einstellung von dehydrierenden Bedingungen auf der Katalysatorstrecke eine Temperatur zwischen 570 und 650°C, vorzugsweise zwischen 580 und 620°C verwendet wird.

6. Verfahren nach einem der Ansprüche 1 bis 5, dadurch gekennzeichnet, daß zur Einstellung von dehydrierenden Bedingungen auf der Katalysatorstrecke eine Raumgeschwindigkeit zwischen 500 und 2000 h$^{-1}$, vorzugsweise zwischen 800 und 1100 h$^{-1}$ verwendet wird.

7. Verfahren nach einem der Ansprüche 1 bis 6, dadurch gekennzeichnet, daß die dehydrierenden Bedingungen über eine Laufzeit zwischen 6 und 100 h, vorzugsweise zwischen 30 und 80 Stunden beibehalten werden.

8. Verfahren nach Anspruch 7, dadurch gekennzeichnet, daß die Katalysatorstrecke zeitlich vor und nach der Dehydrierlaufzeit regeneriert wird.

9. Verfahren nach Anspruch 8, dadurch gekennzeichnet, daß für einen kontinuierlichen Betrieb über die Laufzeit der Katalysatorstrecke hinaus mindestens eine weitere Katalysatorstrecke verwendet wird, wobei im zyklischen Wechsel ständig mindestens eine Katalysatorstrecke unter dehydrierenden Bedingungen betrieben wird und dabei eine weitere Katalysatorstrecke regeneriert wird.

10. Verfahren nach Anspruch 9, dadurch gekennzeichnet, daß für den kontinuierlichen Betrieb insgesamt drei Reaktorstrecken verwendet werden, wobei im zyklischen Wechsel ständig zwei Katalysatorstrecken unter dehydrierenden Bedingungen betrieben werden und dabei eine dritte Katalysatorstrecke regeneriert wird.

11. Verfahren nach einem der Ansprüche 8 bis 10, dadurch gekennzeichnet, daß das Regenerieren der Katalysatorstrecke durch Abbrennen von während der Dehydrierung gebildetem Koks vorgenommen wird, indem ein Regeneriergas über die Katalysatorstrecke geleitet wird, wobei das Regeneriergas durch gleitend oder in Stufen erhöhte Sauerstoffzufuhr, beispielsweise durch Zuspeisung von Luft oder Sauerstoff, zu Wasserdampf und/oder zu einem Inertgas wie z.B. Stickstoff gebildet wird, und indem in einem nachfolgenden Schritt Wasserstoff oder eine Mischung mit Wasserstoff und mit Alkanen über die Katalysatorstrecke geleitet wird, wobei z.B. Alkane wie im Einsatz verwendet werden.

12. Verfahren nach einem der Ansprüche 1 bis 11, dadurch gekennzeichnet, daß in dem die Alkane enthaltenden Einsatz Propan verwendet, in der Katalysatorstrecke hauptsächlich zu Propen umgesetzt, als aus der Katalysatorstrecke austretender Rohpropenstrom verdichtet, in einer $C_2/C_4$-Trennsäule rektifikatorisch in ein $C_{3-}$-Kopfprodukt und ein $C_{3+}$-Sumpfprodukt zerlegt und anschließend das $C_3$-Kopfprodukt durch stufenweise Kondensation in eine $C_2/C_3$-Fraktion und eine hauptsächlich Wasserstoff enthaltende Fraktion zerlegt und die $C_2/C_3$-Fraktion weiter rektifikatorisch in eine $C_{2-}$-Fraktion und eine erste $C_3$-Fraktion zerlegt wird.

13. Verfahren nach Anspruch 12, dadurch gekennzeichnet, daß das $C_{3+}$-Sumpfprodukt rektifikatorisch in eine $C_{4+}$-Fraktion und eine zweite $C_3$-Fraktion zerlegt wird.

14. Verfahren nach Anspruch 13, dadurch gekennzeichnet, daß die erste und die zweite $C_3$-Fraktion einem $C_3$-Splitter zugeführt und in eine Propenfraktion und eine Propanfraktion getrennt werden.

15. Verfahren nach Anspruch 12, dadurch gekennzeichnet, daß das $C_{3+}$-Sumpfprodukt dem $C_3$-Splitter zugeführt wird und die Abtrennung der $C_{4+}$-Komponenten aus dem Sumpfprodukt des $C_3$-Splitters erfolgt.

16. Katalysatorstation mit mindestens zwei Reaktoren zur katalytischen Dehydrierung von Alkanen, wobei jeder Reaktor parallel geschaltete Rohre mit Katalysatorschüttungen enthält und die parallel geschalteten Rohre in einem Feuerraum mit Deckenbefeuerung angeordnet sind, dadurch gekennzeichnet, daß die Katalysatorschüttungen Katalysatorpartikel mit Platin und Zinn auf einem Hydrotalcit-Träger enthalten.

BI.

<u>Figur</u>

Europäisches
Patentamt

**EUROPÄISCHER RECHERCHENBERICHT**

Nummer der Anmeldung

EP 99 12 5259

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (int.Cl.7) |
|---|---|---|---|
| X | WO 94 29021 A (NORSKE STATS OLJESELSKAP) 22. Dezember 1994 (1994-12-22) * Seite 3 - Seite 6 * | 1-16 | C07C5/333 B01J23/62 |
| | | | **RECHERCHIERTE SACHGEBIETE (Int.Cl.7)** |
| | | | C07C B01J |

Der vorliegende Recherchenbericht wurde für alle Patentansprüche erstellt

| Recherchenort | Abschlußdatum der Recherche | Prüfer |
|---|---|---|
| DEN HAAG | 24. März 2000 | Van Geyt, J |

## ANHANG ZUM EUROPÄISCHEN RECHERCHENBERICHT
## ÜBER DIE EUROPÄISCHE PATENTANMELDUNG NR.

EP 99 12 5259

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-03-2000

| Im Recherchenbericht angeführtes Patentdokument | Datum der Veröffentlichung | Mitglied(er) der Patentfamilie | | Datum der Veröffentlichung |
|---|---|---|---|---|
| WO 9429021 A | 22-12-1994 | NO | 932173 A | 15-12-1994 |
| | | AT | 177971 T | 15-04-1999 |
| | | AU | 7009094 A | 03-01-1995 |
| | | DE | 69417416 D | 29-04-1999 |
| | | DE | 69417416 T | 11-11-1999 |
| | | EP | 0705136 A | 10-04-1996 |
| | | ES | 2128565 T | 16-05-1999 |
| | | NO | 954943 A | 06-12-1995 |
| | | US | 5922925 A | 13-07-1999 |
| | | US | 5817596 A | 06-10-1998 |

EPO FORM P0461

Für nähere Einzelheiten zu diesem Anhang : siehe Amtsblatt des Europäischen Patentamts, Nr.12/82